# FASCICULE DE BREVET EUROPEEN

(11) **EP 0 371 891 B1**
(45) Date de publication et mention de la délivrance du brevet: **18.05.1994**
(21) Numéro de dépôt: 89420464.3
(22) Date de dépôt: 24.11.1989
(51) Int. Cl.: B26D 7/20, B26D 3/12, B26F 1/38

(54) **Dispositif de découpage sur des matériaux tels que les non tissés**
Schneidvorrichtung für nichtgewebte Stoffe
Apparatus for cutting non-woven materials

(30) Priorité: 01.12.1988 FR 8816489
(43) Date de publication de la demande: 06.06.1990
(73) Titulaire: MABOTEX S.A., F-42000 Saint-Etienne (FR)
(72) Inventeur: Marion, Louis, F-Roche la Moliere (FR)
(74) Mandataire: Dupuis, François

(56) Documents cités:
- GB-A- 1 427 560
- GB-A- 2 099 306
- US-A- 1 909 027
- US-A- 3 800 640
- US-A- 3 952 637

## Description

L'invention concerne un dispositif de découpage sur des matériaux tels que les non tissés.

L'objet de l'invention se rattache aux secteurs techniques de la manipulation des produits en bandes et des moyens de coupe de ces produits.

Les compresses en matériau non tissé qui sont employées pour panser les blessures, pour absorber le sang ou pour protéger et isoler des plaies, sont en général réalisées sur des machines assurant notamment à partir de bobines la séparation des bandes nécessaires pour la réalisation de la compresse, puis le ou les pliages de la bande, l'empilage des compresses et enfin l'ensachage.

Pour certaines applications, telles que les soins à donner à des malades trachéotomisés il est intéressant d'éxécuter sur la compresse pliée une incision autorisant le passage du cathéter de dérivation voir GB-A-2 099 306.

Certaines machines actuellement sur le marché réalisent cette incision voir, par exemple, document générique US-A-3 800 640, mais elles n'ont pas un rendement important soit par la conception de l'outil de coupe soit parce qu'elles ne travaillent pas en continu.

D'autre part, la forme de l'incision et la cinématique de la machine ne permettent pas toujours de passer plusieurs formats de compresses.

Un des buts de l'invention est de réaliser en continu sur des compresses pliées de formats divers, une incision dont la forme et les dimensions étudiées permettent le passage de cathéters et autres moyens thérapeutiques de sections diverses.

Pour celà et selon une première caractéristique, le dispositif comprend un couteau circulaire présentant au moins une forme de découpe dimensionnée pour réaliser une incision adaptée à plusieurs formats de compresses ; ledit couteau étant entraîné en rotation en fonction du défilement des compresses pliées ; un galet à positionnement élastique étant monté en dessous du défilement des compresses pour servir de contre-outil.

Selon une autre caractéristique, le poste de découpe est intégré dans une machine et est intercalé entre le poste de pliage des compresses et le poste de conditionnement , au dessus de la nappe de transport des compresses par courroie

Une autre caractéristique se trouve dans le fait que l'incision à la forme d'un Y dont le pied s'étend depuis le bord plié de la compresse jusqu'à sa zone médiane ou sensiblement où se développent les branches du Y.

Selon une autre caractéristique la/ou les formes de découpe du couteau circulaire sont établies sur une bague montée sur un cylindre avec possibilité de réglages longitudinal et transversal par rapport à la nappe de courroies de transport.

Dans une autre caractéristique, le galet ou contre-outil à positionnement élastique en pression contre le couteau, est escamotable pour permettre le passage de compresses sans réaliser d'incision.

Ces caractéristiques et d'autres encore ressortiront de la description qui suit.

Pour fixer l'objet de l'invention sans toutefois le limiter, dans le dessin annexé :

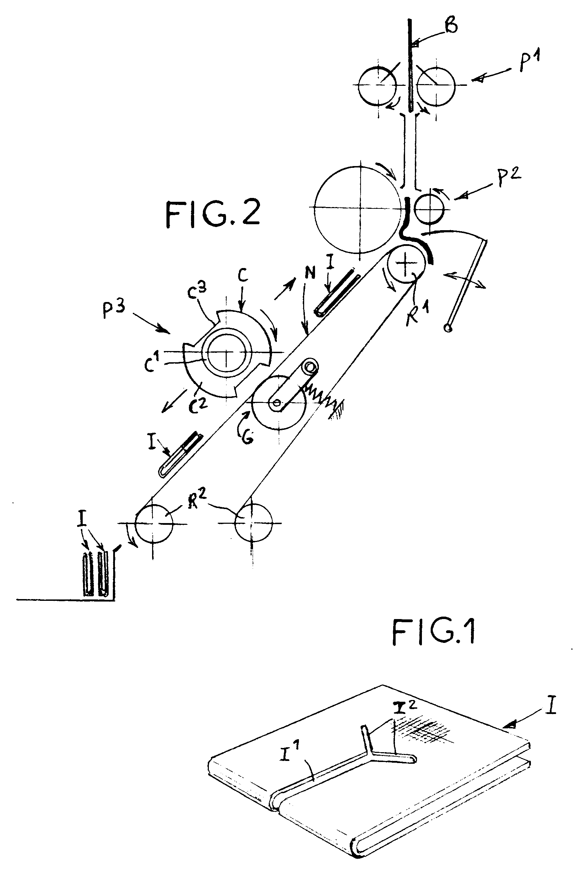

La figure 1 est une vue en perspective illustrant une compresse incisée selon l'invention.
La figure 2 est une vue à caractère schématique montrant le dispositif de découpage intégré dans une machine entre le pliage et le conditionnement des compresses.

Afin de rendre plus concret l'objet de l'invention, on le décrit maintenant, sous une forme non limitative de réalisation illustrée aux figures du dessin.

La figure montre en amont, le poste (P1) de coupe transversale de la bande (B) utilisée pour réaliser les compresses , puis le poste(P2) de pliage de la comrpesse qui peut être à plusieurs ensembles de pliage, le poste (P3) de formation de l'incision sur la compresse pliée et le poste (P4) de conditionnement des compresses.

Le poste (P3) comprend de manière connue une nappe (N) de courroies transporteuses reliées à des rouleaux (R1-R2).

Dans la partie médiane ou sensiblement est monté transversalement au dessus de la nappe un couteau circulaire (C) qui comprend une bague (C1) portant au moins une forme de découpe (C2) et généralement plusieurs pour inciser plusieurs voies de compresses dans la largeur.

La bague est montée sur un cylindre (C3) entrainé en rotation dans un support déterminé par rapport au poste (P1) de coupe transversale.

Les formes de découpe peuvent être réglables transversalement en fonction du format de la compresse et ainsi se trouver toujours entre deux courroies.

De même, le couteau circulaire est réglable longitudinalement en fonction du format de la compresse afin que les formes de découpe pénètrent plus ou moins tôt dans la compresse pliée.

L'invention s'opère bien entendu grâce à un contre outil constitué par un ou plusieurs galets (G) montés dans la nappe en rappel élastique contre le couteau.

Si l'on ne veut pas réaliser une incision dans les compresses défilant sur la nappe, une commande annexe bien connue permet d'escamoter les galets en retrait de la nappe.

Selon une réalisation préférée, quoique non limitative, les formes de découpe sont établies pour réaliser une incision (I) en forme de Y avec le pied (I1) s étendant à partir du bord plié de la compresse et les branches (I2) se développent dans la zône médiane.

Il n'est pas exclu que le dispositif de découpe selon l'invention soit indépendant de toute machine, et dans ce cas on prévoit en amont un poste de distribution des compresses pliées et en aval un poste de réception des compresses incisées.

Les avantages ressortent bien de la discription , on souligne encore la réalisation en continu des incisions, leur forme et leur mode d'actionnement adaptables à plusieurs formats de compresses.

## Revendications

1. Dispositif de découpage sur des matériaux tels que des non tissés, du type rotatif à l'aide d'un couteau circulaire (C) présentant au moins une forme de découpe (C2) dimensionnée pour réaliser une incision (I) caractérisé en ce que la forme de découpe est établie pour réaliser une incision en forme de Y dont le pied s'étend depuis le bord plié d'une compresse jusqu'à sa zone médiane ou sensiblement où se développent les branches (I2) du Y, en ce que la ou les formes de découpe (C2) du couteau circulaire sont établies sur une bague (C1) montée sur un cylindre (C3) avec possibilité de réglages longitudinal et transversal par rapport à la nappe (N) de courroies de transport, en fonction du format des compresses, en ce que ledit couteau est entraîné en rotation en fonction du défilement des compresses pliées ; en ce qu'un galet (G) à positionnement élastique est monté en dessous du défilement des compresses pour servir de contre-outil.

2. Dispositif selon la revendication 1, caractérisé en ce que le galet (G) ou contre-outil à positionnement élastique en pression contre le couteau, est escamotable pour permettre le passage de compresses sans réaliser d'incision.

3. Dispositif selon la revendication 1, caractérisé en ce que le poste de découpe (P3) est intégré dans une machine et est intercalé entre le poste de pliage (P2) des compresses et le poste de conditionnement (P4), au dessus de la nappe (N) de transport des compresses par courroies.

## Claims

1. Rotary type apparatus for cutting non-woven materials using a circular knife (C) having at least one cutting edge (C2) sized in order to make an incision (I) characterised in that the shape of the cut is devised to make a Y shaped incision the base of which extends from the folded edge of a wad to its middle or essentially to the point where the branches (I2) of the Y diverge, in that the cutting edges (C2) of the circular knife are fixed to a ring (C1) mounted on a cylinder (C3) with a longitudinal and transverse adjustment facility with respect to the web (N) of transport belts depending on the size of the wads, in that the said knife is driven in step with the feed of the folded wads and in that an elastically positioned roller (G) is mounted underneath the wad feed and is used as a counter tool.

2. Apparatus according to Claim 1 characterised in that the elastically positioned roller (G) or counter tool which presses against the knife can be removed so that wads can be fed without making an incision.

3. Apparatus according to Claim 1 characterised in that the cutting station (P3) is an integral part of a machine and is situated between wad folding station (P2) and wad conditioning station (P4) above web (N) of the wad transport belt.

## Patentansprüche

1. Umlaufende Schneidvorrichtung für nichtgewebte Stoffe mit einem Kreismesser (C), das zumindest eine Stanzform (C2) für die Ausführung eines Einschnitts (I) aufweist, dadurch gekennzeichnet, daß die Stanzform so ausgebildet ist, um einen Y-förmigen Einschnitt auszuführen, wobei sich der Fuß des Y vom gefalzten Rand einer Kompresse bis ungefähr in ihren mittleren Bereich erstreckt, von wo die Schenkel (I2) des Y ausgehen; und dadurch gekennzeichnet, daß die Stanzform(en) des Kreismessers auf einem Ring (C1) aufgesetzt ist (sind), der auf einer Walze (C3) angebracht ist und in Abhängigkeit vom Kompressenformat die Möglichkeit zur Längs- und Quereinstellung gegenüber der Bahn (N) des Förderbandes bietet; und dadurch gekennzeichnet, daß das besagte Messer je nach Vorbeilauf der gefalzten Kompressen in Umlauf versetzt wird; und dadurch gekennzeichnet, daß eine elastisch positionierbare Rolle (G) unterhalb der vorbeilaufenden Kompressen angebracht ist, um als Gegenwerkzeug zu dienen.

2. Vorrichtung nach Anspruch 1, dadurch gekennzeichnet, daß die elastisch gegen das Messer positionierte Rolle (G) bzw. das Gegenwerkzeug einziehbar ist, um den einschnittfreien Kompressenvorbeilauf zu ermöglichen.

3. Vorrichtung nach Anspruch 1, dadurch gekennzeichnet, daß die Stanzstation (P3) in eine Maschine eingebaut und zwischen der Kompressenfalzstation (P2) und der Abpackstation (P4) oberhalb der Bahn (N) des Kompressenförderbandes eingefügt ist.
